# EUROPEAN PATENT APPLICATION

(11) **EP 1 872 738 A2**
(43) Date of publication of application: **02.01.2008**
(21) Application number: 07111001.9
(22) Date of filing: 25.06.2007
(51) Int. Cl.: A61B 19/08

(54) **Surgical drape**

(30) Priority: 30.06.2006 GB 0612913
(71) Applicant: Finsbury (Development) Limited, Leatherhead, Surrey KT22 0BA (GB)
(72) Inventor: Foley, Andrew John, Sevenoaks, Surrey TN14 5QJ (GB); Tuke, Michael Anthony, Guildford, Surrey GU1 3TF (GB)
(74) Representative: Smaggasgale, Gillian Helen

(57) **Abstract**

A surgical drape (1) for use in orthopaedic surgery which is to be carried out on or at a bone, the sheet comprising: a sheet sized such that in use it will cover at least a site of an incision in the patient; and an aperture (6) in the sheet, the aperture being movable from a wide position, which in use enables the aperture to pass over the end of the bone, to a narrow position which in use provides a close fit around the bone. For example, a portion (4) may be of resilient material or elasticated, so that the aperture may be dilated and then return to a small size.

## Description

The present invention relates to surgical drapes. More particularly, the present invention relates to surgical drapes for use in orthopaedic surgery.

It is conventional practice to cover exposed portions of a patient during an operation except for the actual surgical site. The patient is covered by a mosaic of sheets, usually of rectangular configuration, of woven fabric material. These may be known as drapes. Whilst, these sheets do offer some protection they suffer from various disadvantages the most important of which is that liquids will pass through the material breaking the sterile barrier. It has therefore been suggested that improved drapes may be produced from alternative materials which may be liquid repellant and may be single use.

Whilst this new generation of drapes offer certain advantages in many surgical techniques, in orthopaedic surgery, where the operation is being carried out on a bone and which may be carried out at a joint, the rectangular drapes of the prior art may be difficult to arrange in such a way that only the required area is exposed. This is due to the requirement to cover areas of the body which may not be horizontal and which may not be flat, such as hips and other joints.

A further difficulty arises because orthopaedic surgery can last many hours and it may be necessary to move the patient or his limbs during the surgical procedure. Since the conventional overlapping rectangular drapes can become dislodged or otherwise moved from their optimum position during the protracted operation, the surgical site can become more exposed than is desirable.

With a view to overcoming these problems, various shaped drapes have been suggested. For example, in US 3930497 a drape is disclosed which has a base sheet of a liquid-repellent material which has a U-shaped fenestration and a layer of adhesive surrounding the fenestration. The drape is designed such that the dimensions of the fenestration can be varied. The adhesive is provided with a plurality of cover strips which permit the drape to be applied in a folded condition to the patient and unfolded with reduced danger of contamination.

An alternative arrangement is described in US 4253451 in which a surgical drape for use in hip or knee surgery is described which is designed to provide a substantially complete seal around an operative site in a one step application. The drape has two main sections which are integrally connected along a main folding line, with one main section being continuous along the main folding line and the second main section being trifurcated into a median flap connected to the main folding line along a narrow junction and a pair of side flaps connected to the main folding line along wide junctions. The median flap extends substantially equidistantly from the main folding line as the continuous main section extends therefrom in the opposite direction, while the pair of side flaps extend from the main folding line about midway of the median flap. The median flap is provided about its periphery with a strip of surgical adhesive and the pair of side flaps have a strip of surgical adhesive about their edges adjacent the median flap. The trifurcated main section is folded on the first continuous section and is then positioned, with the median flap under a limb of the patient so that its narrow connecting junction is adjacent the edge of the operative site. Then the median flap and the pair of side flaps are wrapped about the limb so as to provide a substantially complete seal around the operative site with the median flap surrounding one part and the pair of side flaps surrounding the remaining part of the operative site, which is the only part of the limb left exposed.

While these drapes offer solutions over conventional arrangements there is a need for a drape which will provide the required level of protection during orthopaedic surgery, particularly surgery which is carried out at the end of the bone, but which is also simple to use and cost-effective to manufacture.

In order to understand the requirements of a drape for use in orthopaedic surgery, such as hip replacement surgery, it is useful to look at the surgical steps involved in the operation.

In hip replacement operations, the hip joint is exposed and dislocated to provide access to the femoral head. The surgeon may then remove the femoral head and hollow out the femur using reamers and rasps such that it can accept a prosthesis which comprises a head component attached to a stem component via a neck. In use the stem component is inserted into the prepared hollow in the femur.

In an alternative operative technique, the core of the natural head is retained and the prosthesis serves to resurface the femoral head. In femoral head resurfacing techniques, the head of the femur is shaped to fit within the cavity of the resurfacing prosthesis. This generally involves a number of shaping steps including the removal of the dome of the femoral head by means of a saw. A well is usually drilled into the head of the femur either before or after the dome is removed to receive the support pin of the femoral resurfacing prosthesis. Cylinder cutters are then generally used to shape the femoral head until it has a cylindrical profile and at least the lower edges of the cylinder may then be chamfered.

As bone fragments and particles may be produced during the reaming, cutting, drilling and chamfering steps, it is important that the drapes used are able to protect the surgical site from this surgical debris. However, because of the need to work on the end of the bone and to move it around during the techniques, conventional drapes do not generally provide the required level of protection.

In addition, when the prosthesis, whether a full femoral head replacement or a head resurfacing replacement, is inserted onto the femur, cement is usually used to ensure that the prosthesis is held in position. For example, where a head resurfacing prosthesis is used, the resected bone surface, or more usually, the underside of the prosthesis is coated with cement. However, as the prosthesis is a close fit on the bone, as the prosthesis is driven onto the head, excess cement is squeezed out from around the skirt of the head prosthesis. Similarly, as the stem portion of a full femoral prosthesis is driven into the hollow in the resected femur, excess cement may be squeezed out of the top of the bone. Escaping cement can flow down the bone and enter into the surgical site where its presence may lead to post-operative infection.

Similar problems are encountered where the orthopaedic surgery is to be carried out on other bones and/or joints.

It is therefore desirable for drapes to be provided which can be used in surgery of this type and which will effectively prevent bone fragments and cement from entering the body. It is also desirable that the drape can be put in place before machining of the bone commences, so for example, where the operation to be carried out on the femoral head, the drape should be in place before the machining of the head is commenced.

According to the present invention there is provided a drape for use in surgery, the drape comprising: a sheet sized such that in use it will cover at least a site of an incision in the patient; and an aperture in the sheet, the aperture being movable from a wide position, which in use enables the aperture to pass over the end of the bone, to a narrow position which in use provides a close fit around the bone.

The drape of the present inventions is particularly useful in surgery which is to be carried out at, or near, the end of the bone. It will be understood that the phrase the "end of the bone" means either the natural end of the bone such as the end of the femur to an "end" formed along the length of a bone such as by a fracture.

Thus, for example, in hip surgery once the hip joint has been dislocated, the aperture in the drape in its wide position can be passed over the femoral head. The aperture is then moved or moves to the narrow position such that the drape is located around and preferably against the bone. In one arrangement the aperture is provided in the narrow position and is movable to the wide position to allow its application to the bone before being returned to the narrow position.

By "close fit" is meant that the drape is sufficiently close to the bone that cement and other debris will not pass between the edge of the aperture and the bone. In one arrangement, the narrow position provides a tight fit around the bone.

Thus, once in position, as the sheet forms a continuous drape around, and preferably against the bone, debris, such as bone fragments and cement, is prevented from passing between the bone and the drape and into the surgical site. Thus the drape of the present invention not only provides the general protection afforded by the prior art devices, but also provides an arrangement in which the drape is held adjacent to the bone and close thereto such that, for example, escaped cement running down the bone as the prosthesis is inserted, is prevented from running further down the bone once the drape is encountered, and is diverted onto the upper surface of the drape.

Whilst the sheet will be sized to at least cover the site of the incision in the patient, it will generally be substantially larger. For example, where the surgery is being carried out on the hip, the sheet may be sufficiently large to extend across the body of the patient and over the sides of the operating table. The surgical drape may be of any suitable shape. In one embodiment, the drape may be circular. The aperture is preferably located at or near the centre of the sheet. In one arrangement, the circular drape may have a diameter of from about 20 cm to about 100 cm. The sheet preferably has an average thickness of from about 0.05 mm to about 1mm.

The sheet may be provided with means for retaining debris. Any suitable means may be used to prevent debris from passing beyond the outer edge of the drape. The retaining means may comprise a pocket located on an upper surface of the drape. Additionally or alternatively, an upturned wall may be provided on at least a portion, preferably around the entirety of the drape. The upturned wall may be located at any suitable position on the drape. In one arrangement, it is located at or near the edge of the drape.

Any suitable means may be provided to enable the aperture to be movable from the wide position to the narrow position. In one arrangement, the material in the region of the sheet around the aperture, enables the aperture to move from the wide position to the narrow position. In this arrangement, the material from which the drape is manufactured may be sufficiently flexible and resilient to expand from the narrow position to the wide position and then return to the narrow position once the aperture has been passed over the bone.

In one alternative arrangement, the portion of the drape around the aperture may be formed from material which provides the required level of flexibility to allow the size of the aperture to be moved as required. Alternatively, separate means, such as elastic material, may be applied to the material of the sheet in the area around the aperture. Where separate means are used, they may be a single component extending around the aperture or a plurality of means spaced around the aperture.

It will be understood that for ease of manufacture, the sheet may be formed of the material required to allow the aperture to be movable from the wide position to the narrow position. However, in another arrangement, the region of the sheet around the aperture, may be formed from different material to the remainder of the sheet. Where different materials are used, they may be connected by any suitable means. In one arrangement, they may be connected by welding.

The aperture may be of any suitable shape but will generally be substantially circular. Where the sheet is not formed totally from material which allows the size of the aperture to be adjusted, the region of the sheet around the aperture may be an annulus of resilient material.

In a further alternative arrangement, the region of the drape around the aperture may be inflexible. In this arrangement the aperture itself may be at the large size and closure means such as tapes may be provided to close the aperture to the small size once the drape is in position.

In a still further alternative arrangement, the means by which the aperture is movable from the wide position to the narrow position, may be a gusset or cut extending from the aperture into the sheet. In this arrangement, suitable fastening means may be included. Any suitable fastening means may be used including adhesive strips or interlocking closures, for example, hook and loop type fasteners such as those sold under the trade mark Velcro, or zips.

The sheet may be formed of any suitable material. In one arrangement, the sheet or a portion thereof may be transparent. Particularly preferred materials are those which are impermeable, non-woven and capable of being sterilised. Examples of suitable materials include plastics materials such as polyurethane or polyethylene.

Once the operation is complete, the drape has to be removed from around the bone. Although the drape may be lifted such that the bone passes back through the aperture, this is not preferred since material such as cement present on the surface of the drape may fall into the surgical sites or be wiped onto the surface of the bone or the inserted prosthesis. Thus the drape is preferably removed by causing the sheet to break from the outer edge thereof to the aperture. This may be achieved by cutting but in a preferred arrangement, the sheet may include at least one line of weakness to facilitate cutting or to allow the sheet to be simply torn.

The line of weakness may be provided by any suitable means. For example, it may be a frangible flange formed by a thinner area of material. In one arrangement, it may be a line of perforations or a tear strip. It will be understood that such perforations must be sufficiently small that the integrity of the sheet is not compromised. Further the line of weakness should be sufficiently strong that the sheet does not tear accidentally during the operation.

Whilst the line of weakness may run from the edge of the sheet to the aperture, this may provide a risk of the inner edge of the sheet rupturing as the aperture is moved from the wide position to the narrow position. Thus in a preferred arrangement, the line of weakness runs from the edge of the sheet to a point spaced from the aperture. In this embodiment, when the operation is completed, the line of weakness can be caused to rupture. As the portion of the drape around the aperture will still be intact, this can then be cut or torn apart.

The surgical drape may comprise an upstanding collar which extends from the edge of the sheet adjacent the aperture. This arrangement further minimises the risk of debris entering the surgical site.

In embodiments where the drape is formed of more than one material, the drape may be formed by welding different materials together.

The surgical drape may include in antimicrobial agent and/or an antibiotic. These materials may be included in any suitable means. The drape may be coated or impregnated with the antimicrobial agent and/or antibiotic.

The aperture in the drape may be of any suitable size. In one arrangement, the aperture may be circular and may have a diameter of from about 1 cm to about 6 cm.

The present invention will now be described by way of example with reference to the accompanying figures in which:
- Figure 1: is a plan view of a drape of the present invention; and
- Figure 2: is a cross-section view of a drape of the present invention in place on a human femur.

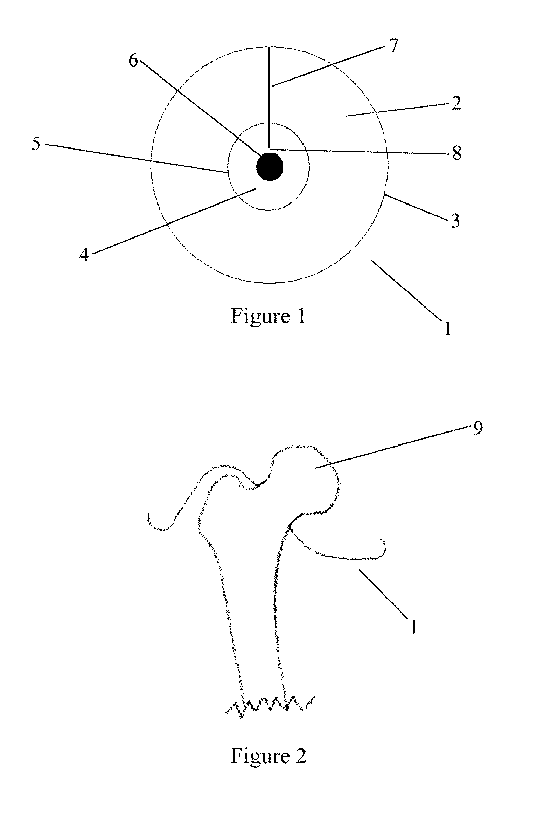

The drape 1 illustrated in Figure 1 is circular. An aperture 6 is located in the centre of the drape I within a resilient portion 4. Extending outwardly from the resilient portion 4 is an outer portion 2. Margin 5 is the boundary between the resilient portion 4 and the outer portion 2. Although in the illustrated embodiment this margin 5 is visible, this may not be the case in practice.

The materials of the resilient portion 4 and the outer region 2 may be different. In this arrangement, a weld will be provided at the margin 5. The outer region 2 is formed of a durable plastics material and the resilient portion 4 is formed of a flexible, resilient plastics material.

A line of weakness 7 runs from the outer edge 3 of the outer region to a position adjacent to the aperture 6. The line of weakness 7 is formed by laser ablation. This line of weakness facilitates easy tearing of the drape 1 once it is no longer required.

However, the line of weakness 7 does not run all the way to the aperture 5; an unweakened portion 8 of the flexible region 4 remains. This is to prevent the line of weakness 7 from being inadvertently torn if the aperture is dilated, e.g. to pass over a femoral head.

A drape 1 of the present invention in place on a human femur is illustrated in Figure 2. In use the drape is presented to the femoral head such that the resilient portion 4 is temporarily dilated so that the aperture 6 can be passed over the femoral head 9. Once the drape is located in the required position on the femoral neck, the resilient portion contracts to the original dimensions which provides a contiguous seal around the femoral neck.

The outer edge 3 of the circumferential region is provided with a lip 11 which runs around the outer edge 3. The purpose of this lip 11 is to prevent any debris which is created during surgery from traveling beyond the outer edge 3 of the circumferential region 2.

Whilst the present invention has been described with particular reference to use in femoral surgery, it will be understood that it can also be used in other forms of surgery, particularly orthopaedic surgery.

## Claims

1. A surgical drape for use in orthopaedic surgery which is to be carried out on or at a bone, the sheet comprising: a sheet sized such that in use it will cover at least a site of an incision in the patient; and an aperture in the sheet, the aperture being movable from a wide position, which in use enables the aperture to pass over the end of the bone, to a narrow position which in use provides a close fit around the bone.

2. A surgical drape according to Claim 1 wherein the aperture is provided in the narrow position and is movable to the wide position to allow its application to the bone before being movable to the narrow position.

3. A surgical drape according to Claim 1 or 2 wherein the drape is circular.

4. A surgical drape according to Claim 3 wherein the drape has a diameter of from about 20 cm to about 100 cm.

5. A surgical drape according to any one of Claims 1 to 4 wherein the aperture is located at or near the centre of the sheet.

6. A surgical drape according to any one of Claims 1 to 5 wherein the sheet has an average thickness of from about 0.05 mm to about 1mm.

7. A surgical drape according to any one of Claims 1 to 6 wherein the sheet is provided with a means for retaining debris.

8. A surgical drape according to Claim 7 wherein the means for retaining debris is a pocket.

9. A surgical drape according to Claim 7 wherein the means for retaining debris is an upstanding wall.

10. A surgical drape according to any one of Claims 1 to 9 wherein the material in the region of the sheet around the aperture, enables the aperture to move from the wide position to the narrow position.

11. A surgical drape according to Claim 10 wherein a region of the sheet around the aperture is formed from different material to the remainder of the sheet.

12. A surgical drape according to any one of Claims 1 to 11 wherein the sheet is formed from plastics materials.

13. A surgical drape according to Claim 12 wherein the plastics material is polyurethane or polyethylene.

14. A surgical drape according to any one of Claims 1 to 13 wherein the sheet includes a line of weakness.

15. A surgical drape according to Claim 14 wherein the line of weakness is a frangible flange, a line of perforations or a tear strip.

16. A surgical drape according to Claim 14 or 15 wherein the line of weakness runs from the edge of the sheet to a point spaced from the aperture.

17. A surgical drape according to any one of Claims 1 to 16 wherein the surgical drape includes an antimicrobial agent and/or an antibiotic.

18. A surgical drape according to any one of Claims 1 to 17 wherein the aperture is circular and has a diameter of from about 1 cm to about 6 cm.
